# EUROPEAN PATENT SPECIFICATION

(11) **EP 2 313 118 B1**
(45) Date of publication and mention of the grant of the patent: **12.03.2014**
(21) Application number: 09785346.9
(22) Date of filing: 16.07.2009
(51) Int. Cl.: A61L 15/22, A61L 15/28, A61L 15/42, A61L 15/60

(54) **COMPOSITIONS FOR USE AS OR IN WOUND DRESSINGS**
ZUSAMMENSETZUNGEN ZUR VERWENDUNG ALS BZW. FÜR WUNDAUFLAGE
COMPOSITIONS POUR UTILISATION EN TANT QUE OU DANS DES PANSEMENTS

(30) Priority: 16.07.2008 GB 0813040
(43) Date of publication of application: 27.04.2011
(73) Proprietor: First Water Limited, Marlborough Wiltshire SN8 2RB (GB)
(72) Inventor: MUNRO, Hugh Semple, Warwickshire GL55 6QT (GB); ANDREWS, Philip, Wiltshire SN8 2RB (GB)
(74) Representative: Brown, David Leslie
(86) International application number: PCT/GB2009/050870
(87) International publication number: WO 2010/007436

(56) References cited:
- EP-A2- 1 649 873
- WO-A1-01/96422
- WO-A2-2007/007115
- US-A- 4 979 946
- US-A- 5 695 777

## Description

The present invention relates to absorbent hydrogel composites, and more particularly to sheet hydrogel composites suitable for use in wound and burn dressings and other applications where absorption of fluid is required. The invention also relates to processes for the manufacture of the novel hydrogel composites, and to uses of the compositions.

The expressions "hydrogel" and "hydrogel composites" used herein are not to be considered as limited to gels which contain water, but extend generally to all hydrophilic gels and gel composites, including those containing organic non-polymeric components in the absence of water.

### Background to the Invention

Many types of dressings are known for the treatment of acute and chronic wounds including gauzes, fibrous sheets, foams, hydrocolloids and gels. Fibrous dressings include Aquacel ®, which is available commercially from ConvaTec ®, and ActivHeal ® Aquafiber ®, available commercially from Advanced Medical Solutions. Aquacel is a non-woven fibrous wound dressing in which the fibres comprise sodium carboxymethyl cellulose. ActivHeal ® Aquafiber ® is a non-woven fibrous dressing in which the fibres comprise calcium/sodium alginate and carboxymethylcellulose. Such fibrous dressings promote wound healing to a certain extent. They are able to absorb liquid exudates from a wound directly into the fibres, initially by capillary action and then by chemical absorbency into the material that forms the fibres. However, there is a limit to the absorbency of these fibrous dressings. It would be advantageous to be able to provide a dressing with similar wound healing properties and of a similar thickness, but with a greater absorbency.

Hydrophilic foam dressings are known for the treatment of wounds. Such foams include hydrophilic polyurethane foams such as medical grade foams available from Rynel, e.g. the 562B medical grade foam, or from Corpura, e.g. Vivo MCF. Such foams are able to absorb liquid exudate by capillary action through their pore structure, but generally release the exudate if compressed, since little, if any, of the exudate is bound into the foam material.

EP-A-0541391, describes hydrophilic polyurethane foams for use as absorbent and wound contacting layers in wound dressings.

It is also known to provide wound dressings in which the wound contacting layer comprises a polyurethane hydrogel material especially suitable for absorbing bodily fluids such as wound exudate. For example, US-A-5160328, describes such a dressing having a wound contacting polyurethane hydrogel layer. The polyurethane gel comprises from 0% to 90% of polyhydric alcohol such as polypropylene glycol, from 6% to 60% by weight of an isocyanate- terminated 5 prepolymer, from 4% to 40% by weight of a polyethylene oxide based diamine, and the balance water. The hydrogel layer is disposed on a support layer that provides mechanical support for the relatively weak hydrogel.

EP-A-0604103, describes processes by which a polymeric hydrogel can be securely adhered to a substrate to form a hydrogel laminate with greatly improved delamination resistance. The laminate is formed by casting onto a polymeric adhesive-coated substrate an aqueous solution of hydrophilic polymer, then exposing this composite to ionizing radiation which cross-links the hydrophilic polymer to form a hydrogel and also induces copolymerisation of the hydrophilic polymer and the adhesive polymer.

US-A-4668564, describes layered materials for use as a hot or cold compress. The materials comprise a layer of substituted urea/urethane hydrogel material bonded to a porous substrate.

WO0245761A1, describes a polyurethane hydrogel wound contact layer laminated to a polyurethane foam layer where the latter provides increased absorbency.

EP-A-0788378, describes a two-layer wound dressing, particularly for medium to highly exuding wounds - comprising a wound contact layer preferably having positive effect on wound healing and second layer of greater hydrophilicity, defined as the rate of exudate absorption, than the first. The wound contact layer can be a fibrous non woven felt and the second layer may comprise a hydrogel, for example chitosan. The disclosed advantage of this dressing is that fluid is removed further from the interface with the wound and that proteins and growth factors absorbed into the wound contacting layer are then subsequently released back into the wound, although no evidence to support this disclosure was provided.

WO 2007/007115 discloses wound dressings comprising a topical hydrogel composition comprising a hydrophilic polymer carrying multiple pendant sulphonyl groups on each polymer molecule. In these wound dressings, generally, the hydrogel contacts the wound directly. Such dressings have been found to be effective in promoting healing of chronic wounds. However, the absorption of wound exudate into the hydrogel is not always as rapid as may be desired. The wound dressings have been found to increase or decrease the concentration of certain ions in the wound bed fluid. This has been found to occur at a rapid rate. It may not always be desirable to increase the ion concentration in a wound at such a rapid rate.

One of the aims of the present invention is to provide at least an alternative to the dressings of the prior art. The present invention may overcome at least one or more of the problems associated with the prior art. The present invention provides a dressing that removes and binds fluid further from the fluid contacting interface than a number of porous dressings of the prior art, whilst being able to modulate the ion concentrations in the fluid external to the dressing (the supernatant) without the need to release previously absorbed material and without the need for the wound facing or contact layer being less hydrophilic than the second layer.

In the following description, the expressions "modulation", "modulator", "modulate" and related expressions shall be considered as equivalent to and interchangeable with "enhancement or inhibition", "enhancer or inhibitor", "enhance or inhibit" and related expressions.

### Chronic Ulcerous Skin Lesions

Chronic skin lesions arise when a skin wound generally fails to follow an appropriate timely healing process to achieve the normal sustained and stable anatomic and functional integrity of the healed tissue. Generally speaking, a skin lesion which has failed to make at least substantial progress towards healing within a period of at least about three months, or which has become stable in a partially healed state for more than about three months, could be categorised as chronic, although even this general guide is not an absolute marker as the age and fitness of the patient, as well as other factors such as diseases or disorders suffered by the patient (for example, circulatory disorders), can significantly lengthen the normal healing process. A skin lesion which is unhealed after at least about six months can be categorised as chronic.

A chronic skin lesion is ulcerous where it involves focal loss of the epidermis and at least part of the dermis.

Malignant or pre-malignant chronic ulcerous skin lesions may arise in connection with a primary cancer of the skin, or with a metastasis to the skin from a local tumour or from a tumour in a distant site. They may be draining or non-draining. They may, for example, take the form of a cavity, an open area on the surface of the skin, skin nodules, or a nodular growth extending from the surface of the skin.

Benign chronic ulcerous skin lesions are not associated with cancer, and include venous leg ulcers, venous foot ulcers, arterial leg ulcers, arterial foot ulcers, decubitus ulcers (e.g. pressure sores, bedsores), post-surgical ulcerous lesions and chronic burn lesions. They may, for example, take the form of a cavity, an open area on the surface of the skin, skin nodules, or a nodular growth extending from the surface of the skin. Typically, they comprise an open granulating area on the surface of the skin.

Chronic ulcerous skin lesions are usually accompanied by other chronic symptoms apart from the failure of the normal healing process. Typical accompanying chronic symptoms include one or more of pain, exudation, malodour, excoriation, spreading of the wound, tissue necrosis, irritation and hyperkeratosis. Such symptoms can be extremely debilitating and embarrassing for patients, and can seriously harm the patient's quality of life. In severe cases, they can require amputation of limbs or even death.

Chronic ulcerous skin lesions can also be categorised according to their exudation. General categorisation is into the three categories "high exudation", "medium exudation" and "low exudation". Exudate management is a particularly difficult task for the caring professional attending to the patient. A balance needs to be struck between the desire to remove exudate to maintain the patient's quality of life at as high a level as possible, and maintenance of an appropriate level of fluid to prevent the lesion becoming too dry or too wet.

### Brief Description of the Invention

The compositions and dressings of the present invention have been surprisingly found to modulate, for example decrease or increase, the concentration of ions in fluids containing ions and ions and proteins, for example protein solutions, for example serum in skin lesions and in particular chronic ulcerous skin lesions, in a manner not found in the individual components of the composite dressing. Additionally the dressings according to the present invention have been surprisingly found to have superior benefits for the healing of wounds than found in the individual components.

In a first aspect, the present invention provides a method of modulating the concentration of dissolved ions in a liquid comprising contacting the liquid with a composition according to claim 1.

In a third aspect, the present invention provides a method of making a composition for the treatment of wounds, the method comprising

associating a first layer, which comprises a porous, optionally hydrophilic material capable of absorbing fluid at least in part by capilliary action, with a second layer as defined in claim 1.

In a fourth aspect, the present invention provides a wound dressing according to claim 14.

In a fifth aspect, the present invention provides the use of a composition of the second aspect in the manufacture of a topical medicament for the treatment of a wound.

### Brief Description of the Drawings

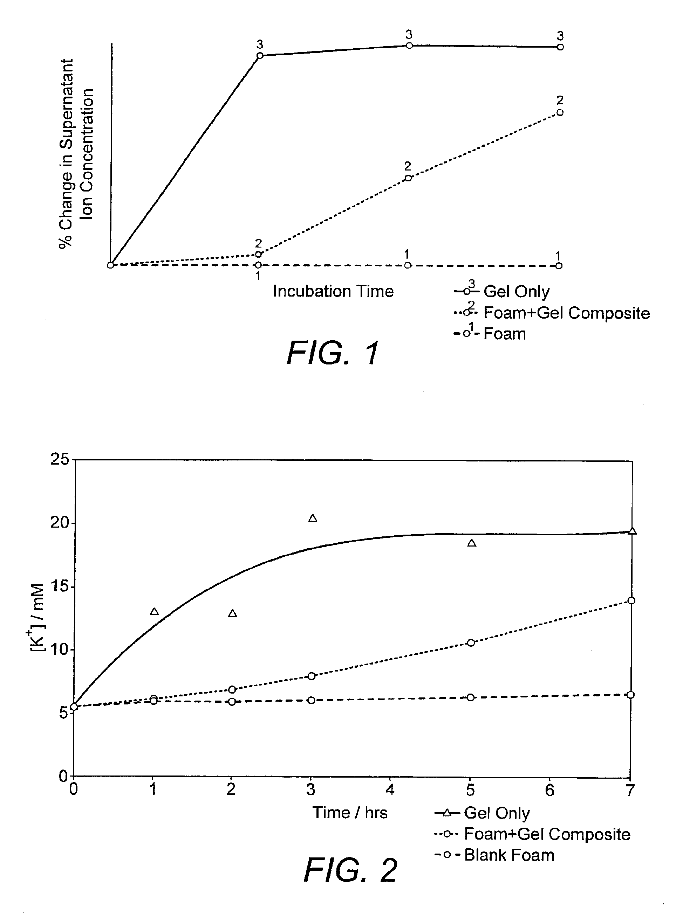

Figure 1, which is merely schematic, illustrates typically how an example composition or dressing of the invention comprising a hydrophilic foam as a first layer and a hydrogel layer as a second layer changes the concentration of an ion in a liquid with which it is contact, e.g. the fluid in a wound, over time, compared to individual components (i.e. the foam and the hydrogel). The change in concentration may be an increase or decrease in concentration. "Incubation time" in Figure 1 simply indicates the time that the dressing and/or individual component thereof is in contact with the liquid.
Figure 2 illustrates the results of an experiment of Example 9 and shows the rate of change in the concentration of potassium ions in a solution in an embodiment of a composition/dressing of the present invention compared to the individual component parts.

### Detailed Description of the Invention

The present invention provides the aspects described above.

Generally, the composition and/or dressing of the present invention comprises a first layer and at least a second hydrogel-containing layer. The first layer is associated with the second layer. In this context, "associated with" preferably indicates that the first layer is in fluid communication with the second layer, i.e. such that fluid can pass from the first layer to the second layer. The first layer may comprise a hydrophilic material and/or a hydrophobic material, preferably a hydrophilic material. Preferably, on contact of the first layer with a liquid (e.g. a wound fluid), the first layer absorbs the liquid by capillary action and transmits some of the liquid to the second layer such that the second layer can absorb at least some of the liquid. The second layer may be in contact with the first layer. One or more further layers or materials may be disposed between the first and second layers. If one or more further layers or materials are disposed between first and second layers, liquid should be able to be transmitted from the first to second layers, e.g. by capillary action.

The composition of the present invention is preferably suitable for use as or in a wound dressing and may be conformable, such that it conforms to the contours within and around a wound.

In the present invention, "wound" includes, but it not limited to, acute and chronic skin lesions and burns.

The first layer may be termed the wound facing layer and may be in direct contact with the wound or have another or several layers interspersed between it and the wound. The first layer is preferably is able to absorb fluid initially at least by capillary forces. The liquid is preferably drawn by capillary forces through at least some of the pores in the first layer and at least some of the liquid may be held within the pores of the first layer. The first layer may comprise a porous matrix, wherein the material of the matrix defining the pores may be able to absorb water, for example the matrix may comprise a hydrogel or hydrocolloid material.

As described herein, the composition and/or dressing of the present invention modulates the concentration of dissolved ions in a liquid (e.g. the fluid in a wound). In this context, "modulates" includes, but is not limited to, a change in the concentration of ions in the liquid (e.g. the fluid in a wound) when comparing the concentration of the ions at a time immediately prior to or on contacting the composition and/or dressing with the liquid and the concentration of the ions in the liquid at a time after contact with the composition and/or dressing with the liquid.

The composition and/or dressing of the present invention may increase or decrease the concentration of dissolved ions in the liquid. The concentration of one or more dissolved alkali metal and/or alkali earth metal ions may be increased or decreased in the liquid. Preferably, the concentration of the one or more dissolved alkali metal and/or alkali earth metal ions is increased in the liquid. The one or more alkali metal ions may be selected from sodium and potassium ions. The one or more alkali earth metal ions may be selected from magnesium and calcium ions.

Preferably, the concentration of dissolved sodium ions and/or dissolved potassium ions is increased in the liquid.

The composition and/or dressing of the present invention may be applied to a wound, for example a chronic ulcerous or acute skin lesion. The first layer should be disposed closer to the wound than the second layer. Accordingly, the liquid may be the fluid in a wound in a human or non-human animal. The liquid may be fluid in a skin lesion for example a skin lesion, for example a chronic ulcerous or acute skin lesion. The fluid in a wound is sometimes termed liquid wound exudate in the art.

The chronic ulcerous skin lesion may be selected from venous leg ulcers, venous foot ulcers, arterial leg ulcers, arterial foot ulcers, decubitus ulcers (e.g. pressure sores, bedsores), post-surgical ulcerous lesions and chronic burn lesions.

Preferably, the second layer has a rate of absorption of fluid that is the same as or less than the first layer. The rate of fluid absorption in the context of the present invention is preferably defined as the amount of fluid absorbed (g/g) by a sample of the relevant layer over a period of 30 minutes, when placed in contact with an aqueous calcium saline solution, as defined in Example 8 below. The method of preparing the sample and measuring the rate of fluid uptake should be in accordance with the method given in Example 8 below. The rate of absorption of the first layer is preferably at least 1.25, preferably, at least 1.5, more preferably at least 2 and even more preferably at least 2.5 times more than the second layer. This is particularly favourable when the absorption capacity of the first and second layers is similar (i.e. within 20% of each other). Suitable materials for use in or as the first layer include, but are not limited to, gauzes, hydrophilic and hydrophobic foams, and gelling fibres, for example alginate and/or carboxymethyl cellulose-containing fibres. Such materials preferably have a higher rate of fluid absorption than the second layer.

The first layer may comprise a hydrophilic, fibrous material and/or a hydrophilic, foamed material. The first layer may comprise a hydrocolloid. The first layer may comprise a hydrophilic, fibrous material, wherein the fibres comprise a hydrocolloid.

The hydrocolloid may comprise one or more of carrageenan, gelatin, pectin, an alkyl cellulose, a carboxyalkyl cellulose, a hydroxyalkyl cellulose, alginic acid, and salts thereof. The hydrocolloid may comprises an alkali metal salt alginate, such as sodium alginate and/or an alkali metal and alkali earth alginate, such as sodium/calcium alginate, as is known in the art. The hydrocolloid may comprises an alkali metal salt of carboxymethyl cellulose, such as sodium carboxymethyl cellulose.

The alkyl cellulose or salt thereof may comprise a C1-C4 alkyl cellulose, for example a methyl or ethyl cellulose, and salts thereof, for example alkali metal salts, e.g. sodium salts, or alkali earth metal salts, such as calcium salts. The carboxyalkyl cellulose or salt thereof may comprise a carboxy C1-C4 alkyl cellulose, such as carboxymethylcellulose and/or carboxyethyl cellulose, and salts thereof, for example alkali metal salts, e.g. sodium salts, or alkali earth metal salts, such as calcium salts. The hydroxyalkyl cellulose or salt thereof may comprise a hydroxy C1-C4 alkyl cellulose, such as hydroxymethyl cellulose or hydroxyethylcellulose, and salts thereof, for example alkali metal salts, e.g. sodium salts, or alkali earth metal salts, such as calcium salts.

"Alkyl" includes, but is not limited to, optionally substituted methyl, ethyl, propyl, and butyl.

Suitable hydrocolloids for use in the invention, for example in the first and/or second layer include, but are not limited to, sodium carboxymethyl cellulose, sodium carboxymethyl 2-hydroxyethyl cellulose, 2-hydroxyethyl cellulose, methyl cellulose, 2-hydroxypropyl methyl cellulose, 2-hydroxyethylmethyl cellulose, 2-hydrobutyl methyl cellulose, 2-hydroxyethyl ethyl cellulose and 2-hydroxypropyl cellulose.

The first layer may comprise a hydrophilic, foamed material comprising a hydrophilic polyurethane foam. Hydrophilic polyurethane foams are known in the art of wound dressings. The hydrophilic polyurethane foam may be as defined in WO 02145761 or EP-A-0541391.

The composition or dressing of the present invention typically has a wound facing face and a non-wound facing face on the opposite side of the composition or dressing. The wound-facing face of the first layer may constitute the wound-facing face of the dressing or at least a part thereof. In one embodiment of the present invention, for example a hydrogel comprising a hydrophilic polymer carrying multiple pendant sulphonyl groups on each polymer molecule, is substantially absent (e.g. having 5 g or less of hydrogel per square meter per face, preferably 2 g or less per square meter per face, more preferably 1 g of less per square meter per face) or absent from the wound facing face of the dressing or composition and/or the wound facing face of the first layer.

In an alternative embodiment, a hydrogel, for example a hydrogel comprising a hydrophilic polymer carrying multiple pendant sulphonyl groups on each polymer molecule, is present on the wound facing face of the dressing or composition and/or the wound facing face of the first layer.

The composition or dressing of the present invention may comprise an absorbent material as disclosed in EP 1649873 A2. This document discloses an absorbent material that comprises a flexible, skin-conformable, moisture-absorbent sheet member, optionally a net member in sheet form overlying and associated with the absorbent sheet member on at least one face thereof, and a hydrogel disposed on at least one of the net member, when present, and the absorbent sheet member in an amount of less than about 500 g of hydrogel per square metre per face, wherein the aqueous saline absorbency rate of the absorbent material through the face on which the hydrogel is disposed is less than about 300 seconds. The first layer of the present invention may comprise an absorbent sheet member as defined in EP 1649873 A2. The first layer of the present invention may comprise a fibrous absorbent sheet member as described in any part of any one of paragraphs [0031] to [0039] of EP 1649873 A2. The first layer of the present invention may comprise an absorbent sheet member as described in any part of any one of paragraphs [0040] to [0044] of EP 1649873 A2. The first layer of the present invention may comprise a net member as described in any part of any one of paragraphs [0052] to [0060] of EP 1649873 A2.

The first layer may comprise an absorbent material having a hydrogel coated on the liquid or wound facing face thereof. Preferably, the hydrogel coating is such that it includes a plurality of apertures to allow rapid fluid transmission through to the first layer. The first layer may comprise an absorbent sheet member as defined in EP 1649873 A2 having hydrogel on the wound facing face thereof. The hydrogel may be present in an amount of less than about 500 g of hydrogel per square meter of the wound facing face. The hydrogel may be applied to the wound facing face of the composition/dressing and/or first layer by means know in the art, for example by the method as described in EP 1649873 A2, for example any of the methods described in any part of any one of claims 1 to 21 of this document and/or any of the methods described in any part of any one of paragraphs [0061] to [0070], and in any of the Examples 1 to 10 of this document. An advantage of using such an absorbent composition is that the coating of the hydrogel does not occlude the pores of the porous absorbent sheet, and therefore allows rapid fluid transmission through to the pores and the interior part of the absorbent sheet.

As described herein, the second layer comprises an absorbent hydrogel. The hydrogel of the second layer may be in the form of an essentially continuous layer, which includes, but is not limited to, an unbroken layer.

The hydrogel of the second layer may be substantially non-porous, i.e. containing few or no pores. A "substantially non-porous" material includes, but is not limited to, a material having a porosity of 0.1 or less, preferably 0.05 or less, more preferably 0.01 or less. The second layer and/or the hydrogel of the second layer may be relatively non-porous, in relation to the first layer. The second layer may have a porosity that is less than the first layer. Porosity of a material is a known measurement in the field and represents the ratio of the volume of void space of the pores to the total volume of the material. The second layer is able to absorb water into the hydrogel material.

The second layer and/or the hydrogel of the second layer may be partially hydrated. A partially hydrated material has some water-content, but is able to absorb more water. This is advantageous in a wound dressing, as it will maintain a moist atmosphere in and around the wound, but is still able to absorb liquid exudate from the wound. Prior to application of the dressing to the wound, the hydrogel of the second layer may contain at least 10% by weight water, preferably at least 20% by weight water, preferably at least 30% by weight water, optionally at least 50% by weight water. Preferably the hydrogel of the second layer contains of from 25 to 45 % by weight water.

The hydrogel comprises a hydrophilic polymer carrying multiple pendant sulphonyl groups on each polymer molecule. In the hydrophilic polymer at least some of the pendant groups may be present in salt form, so that charge-balancing countercations other than H+ are present in the hydrogel associated with the pendant groups. The hydrogel preferably comprises a polymer formed from the polymerisation of one or more monomers selected from (i) 2-acrylamido-2-methylpropane sulphonic acid and salts thereof and (ii) acrylic acid (3-sulphopropyl) ester and salts thereof. Such salts may be as described herein.

The second layer comprises an absorbent hydrogel, preferably possessing intrinsic therapeutic properties. The second layer and/or the hydrogel of the second layer may have an integral reinforcing fabric or scrim, which may be as described herein. The second layer preferably has a lower rate of fluid absorption than the first layer as described herein.

The second layer may have a fluid absorbency (absorption capacity) that is the approximately the same as or more than the first layer. The fluid absorbency of the first and second layers may be the amount of fluid absorbed into a sample of the layer (g/g), as measured according to the method as defined in Example 7 below, i.e. the amount of fluid absorbed on contacting the sample with a calcium chloride/saline solution over a period of 24 hours under the conditions given in this Example. The fluid absorbency of the first layer, as measured using the method as given in Example 7, may be of from 5 to 30 g of fluid per g of the first layer (which may be defined as 5 to 30 g/g), preferably of from 10 to 25 g/g, more preferably from 12 to 20 g/g. The fluid absorbency of the second layer, as measured using the method as given in Example 7, may be of from 5 to 30 g of fluid per g of the second layer (which may be defined as 5 to 30 g/g), preferably of from 10 to 25 g/g, more preferably from 12 to 20 g/g. The fluid absorbency of the second layer, as measured using the method as given in Example 7, may be within 5 g/g of the fluid absorbency of the first layer (i.e. not out the range +/-5g/g of the absorbency of the first layer), preferably within 4 g, more preferably within 3 g/g.

The absorbent hydrogel second layer may comprise a number of subset layers containing one or more hydrogels, which may be the same as or different from each other and may be the same as or different from the composition of the first layer.

If the first and second layers are laminated together and the second layer comprises a crosslinked hydrogel, the hydrogel has preferably been formed from a pregel mixture comprising one or more monomers and a crosslinking agent, and the weight:weight ratio of the total amount of monomer in the pregel mixture to the amount of crosslinking agent in the pregel mixture is from about 200:1 to about 800:1.

The composition and/or dressing of the present invention may comprise one or more further layers, such as a third layer. Such a third layer may act as a backing layer for the composition or dressing and is disposed on a side of the second layer, which, in use, is disposed away from the liquid or wound. The third layer may be permeable or impermeable to liquid water and/or water vapour. Such backing layers are known in the art. The third layer is preferably able to prevent substantial egress of liquid water from the dressing, but preferably allows the passage of water through the third layer as water vapour, i.e. a breathable material, as known in the art. The third layer preferably has some oxygen and vapour permeability for the transpiration of at least part of the fluid diffusing through the dressing. The third layer may be in direct contact with the second layer.

The third layer may comprise a material selected from polymer films and/or foams and/or fibres, which may be continuous or penetrated with holes. The third layer may comprise a material selected from, for example, polyolefins, polyesters, polyurethanes, carboxymethyl cellulose, hydrocolloids and hydrogels. The third layer may be of larger, smaller or the same surface area than the other layers. When the third layer is of a larger surface area it may include a peripheral skin contacting adhesive on the underside thereof enabling contact with the surrounding wound area.

If the third layer is of the same as or smaller area than the first and/or second layer the dressing may comprise a further fourth layer having a larger surface area than the third layer, wherein the fourth layer may be associated with, e.g. in contact with, the third layer, and the fourth layer may include a peripheral skin contacting adhesive on the underside thereof enabling contact with the surrounding wound area. The fourth layer may comprise a continuous sheet or possess a fenestrated region over the area defined by the other 3 layers. If the third layer is of smaller surface area than the first two layers then further continuous or fenestrated layers maybe used to surround and/or overlay the third layer.

The present invention provides a method of making a composition for the treatment of wounds, the method comprising
associating a first layer, which comprises a porous, optionally hydrophilic material capable of absorbing fluid at least in part by capilliary action, with a second layer comprising the absorbent hydrogel of claim 1 to form the composition. The method may involve associating the first and/or second layers with one or more further layers, e.g. as described herein, for example the third layer. "Associating" includes, but is not limited to, "contacting and adhering".

The first and second layers may be pre-formed and laminated together to form the composition. The lamination may be by contacting the two layers together, and optionally, no additional adhesive material needs to be applied.

The composition may be formed *in situ* on a wound by associating the first layer with the wound and then overlaying the second layer on the first layer to form the composition, together with any other layers that may be present.

If the hydrogel comprises a crosslinked hydrogel that has been formed from a pregel mixture comprising one or more monomers and a crosslinking agent, and the weight:weight ratio of the total amount of monomer in the pregel mixture to the amount of crosslinking agent in the pregel mixture is preferably about 200:1 or more, more preferably about 220:1 or more, still more preferably more preferably about 250:1 or more. The weight:weight ratio of the total amount of monomer in the pregel mixture to the amount of crosslinking agent in the pregel mixture is preferably 800:1 or less, more preferably 700:1 or less. The weight:weight ratio of the total amount of monomer in the pregel mixture to the amount of crosslinking agent in the pregel mixture is preferably from about 200:1 to about 800:1, more preferably from about 250:1 to about 800:1, still more preferably about 250:1 to about 700:1.

Without wishing to be bound by theory, the composite dressing, particularly when it includes a partially hydrated hydrogel as described herein, is believed to initially remove excess fluid from the wound surface without any specific modulation of the ion content of the supernatant but on further exposure as the partially hydrated hydrogel begins to swell, starts to modulate the concentration of ions in the fluid external to the composite dressing. It is believed that the combination of initially removing fluid from the site of the skin lesion coupled with the time delayed modulation of the ions in the fluid remaining external to the composite dressing results in the enhanced facilitation of wound healing.

As discussed in more detail below, we have shown that the beneficial effects of the composition and/or dressing of the present invention derive from the presence of the hydrogel, a hydrogel comprising a hydrophilic polymer carrying multiple pendant sulphonyl groups, optionally with multiple pendant carboxylic groups, on each polymer molecule. The dressing may act to increase or decrease the concentration of one or more salts in a liquid, e.g. the naturally exuded salts in the fluid in wound, and/or to selectively absorb one or more salts in a liquid, e.g. the naturally exuded salts in the wound bed, without the need for externally applied salt or other ionic aqueous solutions, and preferably also in the absence of salt or other ionic aqueous solutions in the liquid held within the polymer matrix of the hydrogel, so that the blocking mechanism preventing completion of the normal wound healing process is overridden, bypassed, shut off or otherwise disabled, and continuation of the normal wound healing process to substantial completion is enabled or initiated.

The selectivity of the concentration of the naturally exuded salts is preferably achieved through the control of the counterion(s), being present in the hydrogel, e.g. on the sulphonyl groups or present on the multiple sulphonyl and/or carboxylic groups. Generally speaking, it is believed that selection of, say, sodium counterions on -SO₃⁻ groups (i.e. a sulpho group in salt form) will favour concentration of sodium salts (e.g. sodium chloride) in the wound bed, whereas selection of, say, potassium counterions on -SO₃⁻ groups will favour concentration of potassium salts (e.g. potassium chloride) in the wound bed whereas selection of, say, calcium counterions on -SO₃⁻ groups will favour concentration of calcium salts (e.g. calcium chloride) in the wound bed.

Sodium and potassium counterions are present in a molar ratio of the sodium ions to the potassium ions in the range between 100:0.1 and 100:10.

From this, it is now possible to control the healing process in wound without the need for externally applied salts or other bioactive agents apart from the dressing itself, and more particularly without the need for salts or other bioactive agents in the dressing apart from the hydrogel polymer (including the associated water and the ions of the hydrogel polymer) of the dressing itself.

The dressing may be applied for an effective period of time to effect a modulation in the concentration of ions in the liquid, e.g. in the fluid in a wound. The effective period of time will vary from subject to subject, but generally speaking an effective period of time will be the time in which a wound may take to improve and/or heal, and may be up to about 25 weeks, for example between about 3 days and 25 weeks, depending on the seriousness of the wound and whether it is acute or chronic. Regular changes of the dressing will be required, particularly with more serious and exuding wounds.

The composite dressing comprises a wound facing layer (first layer) that is able to absorb fluid initially at least by capillary forces, for example as shown in gauzes, hydrophilic and hydrophobic foams, and gelling fibres, for example alginate and/or carboxymethyl cellulose based fibres. During the initial phase of fluid uptake there is little specific modulation of the supernatant ion concentration. As exposure time increases the concentration of certain ions (those of lower concentration in the supernatant than present for example in the second layer of the dressing) in the supernatant begins to increase or decrease. This behaviour is not observed for the partially hydrated gel component of the composite dressing on its own in direct contact with fluid. As can be seen from the Examples and accompanying figures, there is a rapid initial modulation (increase) of the supernatant ion. For the wound contacting layer of the composite dressing, where for example the material is a hydrophilic foam, there is little or no modulation of the same ion over the same incubation/exposure times.

Generally, the compositions/dressings of the present invention are such that, in use, the hydrogel of the second layer is not in contact with the wound. This, together with the surprising findings associated with the compositions/dressings of the present invention of an ability to absorb liquid wound exudates relatively quickly, while still being able to modulate the concentration of ions in the fluid in the wound, makes effective treatment available to a wider class of patients having a range of wound conditions, including, but not limited to, patients where the direct application of a hydrogel to a wound bed may not be appropriate. Such patients may include those that have an allergic reaction to a hydrogel and/or patients in which the direct application of a hydrogel to a wound may prevent clotting of blood. The present invention is particularly suited to the treatment of medium to heavily exuding wounds, which may be acute or chronic wounds, where the potential anti-coagulant effect of direct contact of certain types of hydrogel could be problematic. The present invention may be used on patients where, for example, the direct application of porous dressings alone (e.g. hydrophilic foam or fibrous dressings) has been ineffective in promoting wound healing.

It has also been found that the extent of adhesion of the (preferably partially hydrated) hydrogel to the wound facing layer can have an important impact on the efficacy of the composite dressing. In one embodiment of the invention the hydrogel is adherent enough to the wound facing layer (first layer) such that on the partially hydrated hydrogel swelling due to the absorption of fluid the partially hydrated hydrogel remains substantially in intimate contact with the wound facing layer and/or other layers/components of the composition/dressing. The adhesion of the hydrogel to the wound contact layer can be controlled by for example adjusting the monomer to crosslinking ratio to achieve a balance between adhesion and gel integrity. Preferably the ratio is greater than 200:1, based on weight, and less than about 800:1, more preferably greater than 250:1 and less than 700:1.

### Construction of the Wound Dressing

The second layer of the wound dressing may comprise one or more layers of material, one or more of which will contain a hydrogel. The absorbent hydrogel is of sufficiently low crosslinker content to minimise hydrogel break up on the absorption of fluid. The amount of crosslinking agent used is such that when the second layer is combined with the other components of the composition/dressing, e.g. the first layer, an acceptable level of adhesion is achieved. The level of crosslinker may be as described herein. It has been surprisingly found that relatively low levels of crosslinker can minimise the break up of the hydrogel and prevent delamination of the hydrogel from the other components in the dressing, e.g. the first layer, whilst still providing a satisfactory level of strength to the dressing. When two or more different hydrogel layers are utilised in the second layer, the layer closest to the wound, in use, is preferably at least 100 microns thick and/or having a weight of about 100g per meter squared. Details of the crosslinker and the amount used are further described herein.

The composition may comprise a second layer having the first layer in direct contact therewith. The first layer may be so positioned by prepolymerising the crosslinked hydrogel and placing/laminating the latter down on to the first layer with light pressure, or by placing/laminating the first layer onto the polymerised hydrogel with light pressure. To those skilled in the art the automation of such procedures will be obvious. Alternatively the first layer may be placed onto the surface of the liquid pregel and then curing the pregel to form the second layer, which will be adhered to the first layer. The preferred method is the lamination of the first layer to the polymerised hydrogel.

Still further, as previously mentioned, the hydrogel composition may be present in the form of a sheet having an integral scrim (e.g. a woven or non-woven fabric, or a net). The scrim material may be present, suitably within any one or more absorbent hydrogel layers that are present in the material or article according to the present invention. Such a scrim material may be formed of a material that is natural in origin, synthetic in origin, or partly natural and partly synthetic. The scrim may suitably be in the form of a net or a woven or non-woven fabric. Preferred scrims include those formed from polyolefins, polyamides, polyacrylates, or polyesters, for example non-wovens, foams or nets. The scrim material may, for example, comprise sodium polyacrylate fibres, such as those commercially available under the tradename Oasis™ from Acordis Technical Absorbents Limited. The scrim is preferably provided by introducing it into a laid down (e.g. cast) layer of a pre-gel liquid precursor for the hydrogel layer, before curing, so that the liquid pre-gel covers and surrounds the scrim. On curing of the liquid pre-gel, the hydrogel is thereby formed encapsulating the scrim material. Use of a scrim material in this way is found to be potentially helpful in enhancing the strength and ease of handling of the hydrogel component and/or the finished dressing.

When the hydrogel composition contains water, the water may be present in any suitable amount. The typical range of water content is between 0 and about 95% by weight of the hydrogel. The hydrogel composition may conveniently be classified as "high water content" or "low water content". The expression "high water content" refers particularly to hydrogel compositions comprising more than about 40% by weight of water, more particularly above about 50% by weight, and most preferably between about 60% and about 95% by weight. The expression "low water content" refers particularly to hydrogel compositions comprising up to about 40% by weight of water.

### The Wound Dressing- Physical Parameters

The composite wound dressing may typically have a substantially uniform thickness. The first layer may typically have a thickness in the range of about 0.05 mm to about 10 mm. It may comprise one or more materials selected from a non gelling fibrous material, for example a gauze, a gelling fibrous material, for example Aquacel (ConVatec) or Aquafibre (Advanced Medical solutions) and a hydrophilic foam, for example Rynel or Vivo MCF.03 B2 (Corpura). The hydrophilic foam may also comprise a hydrogel within its pores. The absorbent second layer may typically have a thickness in the range of about 0.2 mm to about 10 mm. The second layer preferably has a thickness less than the first layer, preferably at least 2 times less than the first layer, more preferably at least 3 times less than the first layer. The hydrogel of the second layer may suitably be in the form of a sheet having a mean basis weight of gel in the range of about 0.1 kg/m² to about 4 kg/m².

The water activity, which is related to the osmolarity and the ionic strength of the precursor solution (as measured, for example, by a chilled mirror dewpoint meter, Aqualab T3) of the hydrogel, is preferably between 0.05 and 0.99, more preferably between, 0.2 and 0.99, and even more preferably between 0.3 and 0.98, for example between 0.6 and 0.89. The ionic strength of the precursor solution can therefore be used to optimise the hydrogel properties.

The wound dressing may have an area from about 1 cm² to about 900cm², optionally from 4 cm² to 200 cm², depending on the requirements of the application, e.g. the size of the wound. The dressing preferably has an overall thickness of about 0.2 to about 10 mm, preferably of from about 0.3 to about 5 mm.

The fluid absorption capacity of the absorbent second layer will generally be between about 30% and about 20000% by weight. More typically, the absorption capacity of the hydrogel wound dressing will be between about 100% and about 10000% by weight.

### Ingredients of the Hydrogel Composition

The preferred hydrogel composition to be used in the present invention comprises a plasticised three-dimensional matrix of cross-linked polymer molecules, and preferably has sufficient structural integrity to be self-supporting even at very high levels of internal water content, with sufficient flexibility to conform to the surface contours of the human skin.

The hydrogel compositions with which the present invention is concerned generally comprise, in addition to the cross-linked polymeric network, an aqueous plasticising medium. The materials and processing methods used are normally chosen to provide a suitable balance of adhesive and fluid handling properties for the desired application. For further details of the materials and methods of manufacture of individual component parts, please refer to the prior art documents acknowledged herein, as well as standard texts on hydrogels (e.g. "Hydrogels" in Kirk-Othmer Encyclopedia of Chemical Technology, 4th Edition, vol. 7, pp. 783-907, John Wiley and Sons, New York, the contents of which are incorporated herein by reference.

### Monomers

The hydrogel comprises a hydrophilic polymer carrying multiple pendant sulphonyl groups on each polymer molecule. In the hydrophilic polymer at least some of the pendant groups are present in salt form, so that charge-balancing countercations other than H+ are present in the hydrogel associated with the pendant groups.

Particularly preferred monomers include: the sodium salt of 2-acrylamido-2-methylpropane sulphonic acid, commonly known as NaAMPS, which is available commercially at present from Lubrizol as either a 50% aqueous solution (reference code LZ2405) or a 58% aqueous solution (reference code LZ2405A); the potassium salt of 2-acrylamido-2-methylpropane sulphonic acid (Potassium AMPS), which is available commercially at present from Lubrizol; the ammonium salt of 2-acrylamido-2-methylpropane sulphonic acid (Ammonium AMPS), which is available commercially at present from Lubrizol; acrylic acid (3-sulphopropyl) ester potassium salt, commonly known as SPA or SPAK (SPA or SPAK is available commercially in the form of a pure solid from Raschig); acrylic acid (3-sulphopropyl) ester sodium salt, commonly known as SPANa (SPANa is available in the form of a pure solid from Raschig); and SPDA. Acrylic acid (BASF) may be used as supplied or in partial or complete salt form where the salt counterion is an alkali metal (e.g. sodium or potassium), alkaline earth metal (e.g. calcium) or ammonium. Mixtures of any two or more of the above monomers may be used. When a mixture of the monomers is used, it may, for example, be a mixture of NaAMPS and SPAK, a mixture of NaAMPS and SPANa, a mixture of NaAMPS and Potassium AMPS, a mixture of NaAMPS and Ammonium AMPS, or a mixture of NaAMPS and acrylic acid. The relative amounts of the monomers in a mixture may be dictated by the desired ratio of counterions (e.g. potassium, sodium and ammonium) in the hydrogel, as well as the required properties of the copolymer, and may be selected easily by those skilled in the art, if necessary with routine testing of the copolymers prepared.

The hydrogel may comprise a hydrophilic copolymer formed from a first monomer and a second monomer, wherein the first monomer comprises an olefinically unsaturated sulphonic acid monomer or salt thereof, preferably an acrylic acid ester sulphonic acid monomer or salt thereof, and the second monomer comprises an olefinically unsaturated sulphonic acid monomer or salt thereof, different from the first monomer and preferably an acrylamide sulphonic acid monomer or salt thereof.

The weight ratio (w/w) of the first monomer/second monomer in the hydrogel may be equal to or more than 1 and/or either (i) the sulphonic group in both first and second monomers may be in acidic form or (ii) the sulphonic group in both first and second monomers may be in salt form and the counterion for both monomers is the same.

The weight ratio (w/w) of the first monomer/second monomer in the hydrogel may be equal to or less than 1 and/or either (i) the sulphonic group in both first and second monomers may be in acidic form or (ii) the sulphonic group in both first and second monomers may be in salt form and the counterion for both monomers is the same.

The sulphonic group in both first and second monomers is in salt form and the counterion for both monomers is the same as or different from each other. Both first and second monomers may be salts of olefinically unsaturated sulphonic acid monomers. The counterion for both salts may be the same or different. If the counterion is the same, preferably it is sodium.

The hydrogel may comprise a polymer formed from first and/or second monomers described below. The hydrogel may be a co-polymer of the first and second monomers. The first monomer preferably comprises a compound of formula (I) 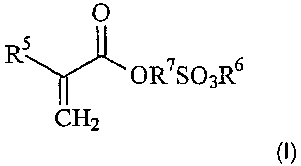 wherein R⁵ represents hydrogen or optionally substituted alkyl, preferably methyl or ethyl, R⁶ represents hydrogen or a cation and R⁷ represents an optionally substituted alkylene moiety, preferably of 1 to 4 carbon atoms. Preferably R⁷ represents optionally substituted n-propyl. Unless otherwise indicated, the term "alkyl", as used herein includes saturated monovalent hydrocarbon radicals having straight or branched moieties, preferably containing 1 to 4 carbons. Examples of alkyl groups include, but are not limited to, methyl, ethyl, propyl, isopropyl, and t-butyl. Unless otherwise indicated, the term "alkylene", as used herein, includes a divalent radical derived from straight-chain or branched alkane. Examples of alkylene radicals are methylene, ethylene (1,2-ethylene or 1,1-ethylene), propylene, trimethylene (1,3-propylene), tetramethylene (1,4-butyfene), pentamethylene and hexamethylene.

The second monomer preferably comprises a compound of formula (II) 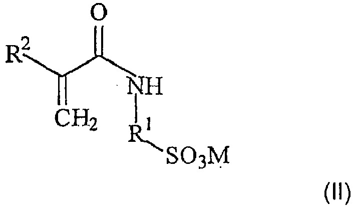 wherein R¹ is an optionally substituted hydrocarbon moiety, R² is hydrogen or optionally substituted methyl and ethyl, and M represents hydrogen or a cation.

R¹ is preferably an optionally substituted alkylene, cycloalkylene or an aromatic moiety. Preferably R¹ represents a saturated moiety or an aromatic moiety. R¹ preferably contains from 3 to 12 carbon atoms, more preferably from 3 to 6 carbon atoms. A preferred moiety which R¹ represents is 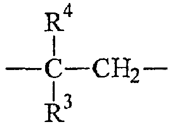 wherein R³ represents hydrogen or an optionally substituted straight or branched chain alkyl group possessing from 1 to 6 carbon atoms and R⁴ represents an optionally substituted straight or branched chain alkyl group possessing from 1 to 6 carbon atoms.

R¹, R², R³, R⁴, R⁵ and R⁷ are optionally substituted by a group which preferably has a tendency to increase the water solubility of the compound. Suitable groups will be well known to a person of skill in the art. A preferred optional substituent is a hydroxyl, amino or ammonium group or a halogen (e. g. chlorine, bromine, or iodine) atom. A suitable cation is an alkali metal cation, especially sodium or potassium.

Most preferably, the first monomer comprises an acrylic acid (3-sulphopropyl) ester or a salt thereof, e.g. an alkali metal salt such as a sodium or potassium salt, of an analogue thereof. A particularly preferred example is acrylic acid (3-sulphopropyl) ester sodium salt, which may be termed NaSPA or SPANa (available in the form of a solid from Raschig).

Most preferably, the second monomer comprises 2-acrylamido-2-methylpropanesulphonic acid or a salt thereof, e. g. an alkali metal salt such as a sodium or potassium salt. A particularly preferred example is the sodium salt of 2-acrylamido-2-methylpropanesulphonic acid (available commercially at present from Lubrizol as a 58% aqueous solution).

Optional substituents of the monomers used to prepare the hydrogels used in the present invention may preferably be selected from substituents which are known in the art or are reasonably expected to provide polymerisable monomers which form hydrogel polymers having the properties necessary for the present invention. Suitable substituents include, for example, lower (C1 to C6) alkyl, hydroxy, halo and amino groups.

### Cross-linking Agents

Conventional cross-linking agents are suitably used to provide the necessary mechanical stability and to control the adhesive properties of the hydrogel. The amount of cross-linking agent required to produce a crosslinked absorbent hydrogel for use in or as the second layer, which is resistant break up and delamination from other layers on the absorption of fluid is from about 0.08 to 0.17% by weight and more preferably between 0.11 and 0.16% by weight of the total polymerisation reaction mixture. The pregel mixture may comprise one or more monomers and a crosslinking agent, and the weight:weight ratio of the total amount of monomer in the pregel mixture to the amount of crosslinking agent in the pregel mixture is preferably 200:1 or more and/or 800:1 or less; preferably the ratio is 200:1 or more and 800:1 or less, more preferably 250:1 or more and 700:1 or less.

Typical cross-linkers include tripropylene glycol diacrylate, ethylene glycol dimethacrylate, triacrylate, polyethylene glycol diacrylate (polyethylene glycol (PEG) molecular weight between about 100 and about 4000, for example PEG400 or PEG600), and methylene bis acrylamide. When two or more hydrogel layers are employed in the absorbent hydrogel second layer of the wound dressing, the amount of cross-linking agent in the different hydrogel layers may be approximately the same, but preferably, the amount of crosslinking agent in the hydrogel layer closest to the wound, in use, has a lower amount of cross linking agent than the one or more further hydrogel layers disposed further away from the wound, to prevent delamination of the second layer from the first layer or other layers disposed between the first and second layers.

### Organic Plasticisers

The pregel mixture for the hydrogel may comprise one or more plasticisers, e.g. an organic plasiticiser. The one or more organic plasticisers, when present, may suitably comprise any of the following either alone or in combination: at least one polyhydric alcohol (including, but not limited to, honey, glycerol, polyethylene glycol, or sorbitol), at least one ester derived therefrom, at least one polymeric alcohol (such as polyethylene oxide) and/or at least one mono- or poly-alkylated derivative of a polyhydric or polymeric alcohol (such as alkylated polyethylene glycol). Glycerol is the preferred plasticiser. An alternative preferred plasticiser is the ester derived from boric acid and glycerol. When present, the organic plasticiser may constitute up to about 60% by weight of the hydrogel composition.

### Surfactants

Any compatible surfactant may optionally be used as an additional ingredient of the hydrogel composition. Surfactants can lower the surface tension of the mixture before polymerisation and thus aid processing. Non-ionic, anionic and cationic surfactants are preferred. The surfactant ideally comprises any of the surfactants listed below either alone or in combination with each other and/or with other surfactants. The total amount of surfactant, if present, is suitably up to about 10% by weight of the hydrogel composition, preferably from about 0.05% to about 4% by weight.

### Other Additives

The hydrogel in the composite of the present invention may include one or more additional ingredients, which may be added to the pre-polymerisation mixture or the polymerised product, at the choice of the skilled worker. Such additional ingredients are selected from additives known in the art, including, for example, water, organic plasticisers, surfactants, polymeric material (hydrophobic or hydrophilic in nature, including proteins, enzymes, naturally occurring polymers and gums), synthetic polymers with and without pendant carboxylic acids, electrolytes, osmolites, pH regulators, colorants, chloride sources, bioactive compounds and mixtures thereof. The polymers can be natural polymers (e.g. xanthan gum), synthetic polymers (e.g. polyoxypropylene-polyoxyethylene block copolymer or poly-(methyl vinyl ether alt maleic anhydride)), or any combination thereof. By "bioactive compounds" we mean any compound or mixture included within the hydrogel for some effect it has on living systems, whether the living system be bacteria or other microorganisms or higher animals such as the patient. Bioactive compounds that may be mentioned include, for example, pharmaceutically active compounds for example Phenytoin,, antimicrobial agents, antiseptic agents, antibiotics and any combination thereof. Antimicrobial agents may, for example, include: sources of oxygen and/or iodine (e.g. hydrogen peroxide or a source thereof and/or an iodide salt such as potassium iodide) (see, for example Bioxzyme™ technology, for example in The Sunday Telegraph (UK) 26 January 2003 or the discussion of the Oxyzyme™ system at www.woundsuk.com/posterabstracts2003.pdf); honey (e.g. active Manuka honey); antimicrobial metals, metal ions and salts, such as, for example, silver-containing antimicrobial agents (e.g. colloidal silver, silver oxide, silver nitrate, silver thiosulphate, silver sulphadiazine, or any combination thereof), hyperchlorous acid; or any combination thereof and copper based agents (e.g. salts complexes and/or dispersions)

In the Bioxzyme system, a dressing comprises two hydrogels. One contains glucose based antibacterial compounds and the other contains enzymes that convert the glucose into hydrogen peroxide. When these are exposed to air and contacted together at a wound site, the enzyme-containing gel being adjacent the skin and the glucose-containing gel overlying the enzyme-containing gel, a low level steady flow of hydrogen peroxide is produced, which inhibits anaerobic bacteria. This antibacterial effect can be enhanced by the inclusion of a very low level of iodide (less than about 0.04%) in the hydrogel. The hydrogen peroxide and the iodide react to produce iodine, a potent antimicrobial agent.

Hydrogels incorporating antimicrobial agents may, for example, be active against such organisms as *Staphylococcus aureus* and *Pseudomonas aeruginosa.*

Agents for stimulating the healing of wounds and/or for restricting or preventing scarring may be incorporated into the first or second layers. Examples of such agents include growth factors such as TGF (transforming growth factor), PDGF (platelet derived growth factor), KGF (keratinocyte growth factor, e.g. KGF-1 or KGF-2), VEGF (vascular endothelial growth factor), IGF (insulin growth factor, optionally in association with one or more of IGF binding protein and vitronectin), e.g. from GroPep Ltd, Australia or Procyte, USA (see, e.g. WO-A-96/02270, cell nutrients (see, e.g., WO-A-93/04691), glucose (see, e.g., WO-A-93/10795); an anabolic hormone or hormone mixture such as insulin, triiodothyronine, thyroxine or any combination thereof (see, e.g., WO-A-93/04691); or any combination thereof.

Additional polymer(s), typically rheology modifying polymer(s), may be incorporated into the polymerisation reaction mixture at levels typically up to about 10% by weight of total polymerisation reaction mixture, e.g. from about 0.2% to about 10% by weight. Such polymer(s) may include polyacrylamide, poly-NaAMPS, polyethylene glycol (PEG), polyvinylpyrrolidone (PVP) or carboxymethyl cellulose.

Additional osmolite(s) may be included to modify the osmolarity of the hydrogel. Osmolites may be ionic (e.g. electrolytes, for example salts which are readily soluble in the aqueous phase of the hydrogel to increase the ionic strength of selected cations or anions and hence the osmolarity of the hydrogel). By selecting the ions present in an ionic osmolite, and particularly by selecting the cation so as to correspond or not with cationic counterions in the monomer(s) of the hydrogel, the ionic strength of certain anions (e.g. chloride) can be varied with fine control, without substantially changing the ionic strength of cations already present in very large amounts as counterions of the monomer(s).

Osmolites may be organic (non-ionic), for example organic molecules which dissolve in or intimately mix with the aqueous phase of the hydrogel to increase the osmolarity of the hydrogel deriving from non-ionic species in the aqueous phase. Such organic osmolites include, for example, water-soluble sugars (e.g. glucose and other monosaccharides), polyhydric alcohols (e.g. glycerol and other polyhydroxylated alkanols).

Additive ingredients may serve more than one purpose. For example, glycerol may serve as an organic plasticiser and an osmolite.

The hydrogel may comprise one or more complexing or chelating agents, which may include, but are not limited to, organic poly-carboxylic acids, and includes, but is not limited to, agents that can form complexes with or chelate to one or more metal ions. The complexing agent may be selected from di-, tri- and tetra- carboxylic acids. Preferably, the one or more complexing or chelating agents contain a moiety in which two carboxylic acid groups (CO₂H) or salts thereof are separated by three or four covalent bonds (e.g. three bonds in malic acid: (HO₂C)-CH₂-CH,OH-(CO₂H); four bonds in EDTA: (HO₂C)-CH₂-NR-C H₂-(CO₂H), in which R is the remaining part of the molecule). The complexing or chelating agents may comprise one or more molecules containing one or more primary, seconday or tertiary nitrogens within their structure.

The complexing or chelating agents may include, but are not limited to, EDTA, citric acid, maleic acid, malic acid, and their salts (which include, but are not limited to, sodium and potassium salts). These agents have been found to be effective in controlling any ion exchange that may be associated with the hydrogel composition.

The hydrogel used in the present invention preferably consists essentially of a crosslinked hydrophilic polymer of a hydrophilic monomer and optionally one or more comonomer, together with water and/or one or more organic plasticiser, and optionally together with one or more additives selected from surfactants, polymers, pH regulators, electrolytes, osmolites, chloride sources, bioactive compounds and mixtures thereof, with less than about 40%, for example less than about 10%, by weight of other additives.

For further details of suitable hydrogel material for use in the present invention, and its preparation, please refer to the following publications: PCT Patent Applications Nos. WO-97/24149, WO-97/34947, WO-00/06214, WO-00/06215, WO-00/07638, WO-00/46319, WO-00/65143 and WO-01/96422.

The present invention will be further illustrated in the following non-limiting Examples, with reference to the accompanying drawings, in which:
Figure 2 shows the varying rates of ion modulation of an embodiment of the present invention compared to the individual components thereof, when tested in accordance with the method of Example 9.

It should be noted that Figure 1, which is merely schematic, is described above and does not show the results of any particular experiment carried out herein.

### Examples

In these examples, each of the pre-gel formulations, unless otherwise described, were coated onto Polyurethane Film (Intelicoat 2301) with a 0.3 to 2.6 kg per square metre coat weight and then cured by a medium pressure mercury arc lamp located within a bench top UV curing machine (NUVA-Solo-30)GEW, UK) at a conveyer speed of 7m/minute.

The exposed surface of the cured gel was then laminated to the wound contact layer by simply pressing the cured gel and wound contact layer together.

### Example 1

Pre-gel: 70 parts by weight of 58% aqueous solution of the sodium salt of acrylamidomethyl-propanesulphonic acid (NaAMPS, LZ2405 Lubrizol), 0.5 parts acrylic acid (3-sulphopropyl) ester potassium salt, commonly known as SPA or SPAK (SPA or SPAK is available commercially in the form of a pure solid from Raschig), 30 parts glycerol and 0.21 parts of a 1 to 10 (by weight) mixture of Daracure 1173 photoinitiator (Ciba Speciality Chemicals) and IRR280 cross-linker (PEG400 diacrylate, UCB Chemicals).

### Example 2

Pre-gel: 70 parts by weight of 58% aqueous solution of the sodium salt of acrylamidomethyl-propanesulphonic acid (NaAMPS, LZ2405 Lubrizol), 0.5 parts acrylic acid (3-sulphopropyl) ester potassium salt, commonly known as SPA or SPAK (SPA or SPAK is available commercially in the form of a pure solid from Raschig), 30 parts glycerol and 0.18 parts of a 1 to 10 (by weight) mixture of Daracure 1173 photoinitiator (Ciba Speciality Chemicals) and IRR280 cross-linker (PEG400 diacrylate, UCB Chemicals).

### Example 3

Pre-gel: 70 parts by weight of 58% aqueous solution of the sodium salt of acrylamidomethyl-propanesulphonic acid (NaAMPS, LZ2405 Lubrizol), 0.5 parts acrylic acid (3-sulphopropyl) ester potassium salt, commonly known as SPA or SPAK (SPA or SPAK is available commercially in the form of a pure solid from Raschig), 30 parts glycerol and 0.12 parts of a 1 to 10 (by weight) mixture of Daracure 1173 photoinitiator (Ciba Speciality Chemicals) and IRR280 cross-linker (PEG400 diacrylate, UCB Chemicals).

Example 1, 2 and 3 were laminated to a 3.5mm thick hydrophilic polyurethane foam (Corpura (Holland)Vivo MCF.03 B2). They were then placed on a dynamic wound model (SMTL, Cardiff) and challenged for 24 hours with Hanks Solution (Aldrich) at a dosage of 1ml per hour. The exit pipe of the wound model was sealed. Examples 2 and 3 did not delaminate. Example 1 delaminated and was deemed to be not a preferred embodiment.

### Example 4

Pre-gel: 70 parts by weight of 58% aqueous solution of the sodium salt of acrylamidomethyl-propanesulphonic acid (NaAMPS, LZ2405 Lubrizol), 0.5 parts acrylic acid (3-sulphopropyl) ester potassium salt, commonly known as SPA or SPAK (SPA or SPAK is available commercially in the form of a pure solid from Raschig), 30 parts glycerol and 0.18 parts of a 1 to 10 (by weight) mixture of Daracure 1173 photoinitiator (Ciba Speciality Chemicals) and IRR280 cross-linker (PEG400 diacrylate, UCB Chemicals).

The pre-gel was coated onto a polyester non woven fabric which was itself placed on siliconised release paper at a coat weight of 1.0 kg per square metre. The gel was cured (Gel A). A further pre gel Example 3, was coated onto the cured gel A at a coat weight of 0.4 kg per square metre and cured. This was then laminated to a hydrophilic foam as in the previous examples. The non wound contact side was then laminated to an acrylic adhesive coated polyurethane foam (Inspire 2317).

### Example 5

Pre-gel: 70 parts by weight of 58% aqueous solution of the sodium salt of acrylamidomethyl-propanesulphonic acid (NaAMPS, LZ2405 Lubrizol), 0.5 parts acrylic acid (3-sulphopropyl) ester potassium salt, commonly known as SPA or SPAK (SPA or SPAK is available commercially in the form of a pure solid from Raschig), 30 parts glycerol and 0.12 parts of a 1 to 10 (by weight) mixture of Daracure 1173 photoinitiator (Ciba Speciality Chemicals) and IRR280 cross-linker (PEG400 diacrylate, UCB Chemicals).

### Example 6

Pre-gel: 55 parts by weight of 58% aqueous solution of the sodium salt of acrylamidomethyl-propanesulphonic acid (NaAMPS, LZ2405 Lubrizol), 15 parts acrylic acid (3-sulphopropyl) ester potassium salt, commonly known as SPA or SPAK (SPA or SPAK is available commercially in the form of a pure solid from Raschig), 30 parts glycerol and 0.15 parts of a 1 to 10 (by weight) mixture of Daracure 1173 photoinitiator (Ciba Speciality Chemicals) and IRR280 cross-linker (PEG400 diacrylate, UCB Chemicals).

### Example 7 - Absorption Capacity of component layers

Weighed samples of the component layers (circa 2g) were immersed in 100ml of a saline solution (21 g Sodium Chloride, 0.7g Calcium chloride, 2500g water) for 24 hours at around 25 degrees Celsius. After immersion samples are reweighed and the weight uptake per gram of material calculated.

| Material | Weight Uptake (g/g) |
|---|---|
| Gel (Example 4) | 18.5 |
| Aquacel (ConVatec) | 16.7 |
| Vivo MCF 03 B2 3mm thick (Corpura) | 18.7 |

The data show that the absorption capacity (an indication of hydrophilicity) of all three materials is similar.

### Example 8 - Rate of Fluid (Exudate) uptake

### APPARATUS NEEDED:

Gel - Example
Foam
Gel/Foam Composite
circle template 5cm diameter
Scissors
Balance
Paddington cups (Surgical Materials Testing Laboratory, Cardiff, UK)
Calcium Saline solution (0.81 to 0.85 g NaCl and 0.027 to 0.029 g CaCl₂ and purified water to 100g) use analytical grade anhydrous salts to prepare the isotonic solutions
Torque controlled screwdriver
Tray
Oven at 40°C, 55% RH
Balance

### METHOD:

1. Lay the sample down on a flat surface, and draw a circle using the template. Cut the circle out. Cut two circles for each sample to be analysed.
2. Take a Paddington cup, place on the balance and record the mass.
3. Fill the Paddington cup with 10g of test fluid (Calcium Saline).
4. Remove the lid from the Paddington cup.
5. Remove the liners from one side of the sample, and carefully stick the sample down, ensuring that there are no wrinkles, that the central hole is covered with gel, but that the screw holes are not.
6. Remove the liners from the upper surface of the sample as necessary. Put the lid back on and tighten the screws using the screwdriver until a torque of 40 cN.m is reached. (Very soft fragile samples may not be as tightly fastened as stronger ones - ensure that the samplel has not ripped)
7. Repeat for all samples.
8. When all the Paddington cups have been prepared, place the samples on a tray, in an inverted position such that the fluid directly contacts the dressing, and place the tray in the oven.
9. Record the time at which the samples are placed in the oven.

After 30 minutes hours remove the supernatant fluid, and measure and record the weight. The difference between the original weight of fluid (10g) and the remaining weight of fluid (supernatant) is the weight uptake by the sample

### Results

| Sample | Weight Uptake(g) |
|---|---|
| Corpura Foam Vivo MCF.03 B2 (3mm thick) | 7.6 |
| Gel (Example 4) | 2.6 |

The data show that the amount of fluid absorbed by the foam in 30 minutes is far greater than the gel, hence the foam has a faster rate of fluid uptake.

### Example 9 - Ion Modulation

### APPARATUS NEEDED:

Gel - Example
Foam
Gel/Foam Composite
circle template 5cm diameter
Scissors
Balance
Paddington cups (Surgical Materials Testing Laboratory, Cardiff, UK)
Hanks solution (H9269), available from Aldrich.
Torque controlled screwdriver
Tray
Oven at 40°C, 55% RH
Beckman Synchron Elise Electrolyte System (Potassium Electrode)

### METHOD:

1. Lay the sample down on a flat surface, and draw a circle using the template. Cut the circle out. Cut two circles for each sample to be analysed.
2. Take a Paddington cup, place on the balance and record the mass.
3. Fill the Paddington cup with 20g of test fluid (Hanks solution).
4. Remove the lid from the Paddington cup.
5. Remove the liners from one side of the sample, and carefully stick the sample down, ensuring that there are no wrinkles, that the central hole is covered with gel, but that the screw holes are not.
6. Remove the liners from the upper surface of the gel. Put the lid back on and tighten the screws using the screwdriver until a torque of 40 cN.m is reached. (Very soft fragile gels may not be as tightly fastened as stronger ones - ensure that the gel has not ripped)

Repeat for all samples.
7. When all the Paddington cups have been prepared, place the samples on a tray, in an inverted position such that the fluid directly contacts the dressing, and place the tray in the oven.
8. Record the time at which the samples are placed in the oven.

After 1,2,3,5 and 7 hours remove the supernatant, and measure and record the potassium concentration.

The results are displayed in Figure 2 and demonstrate the modified ion modulation behaviour the of the gel/foam composite dressing.

### Example 10

Patient with a venous leg ulcer of 24 months duration. The wound had been dressed with a variety of dressings during this period. Prior to dressing the wound with a gelling fibre/gel composite of the present invention, the wound had been treated with Aquacel for 10 days. At the point of changing the dressing regime to the composite dressing (comprising 5cmx5cm sheet of Aquacel and a 10cmx10cm sheet of example 3) the wound showed deterioration of the surrounding tissue and the patient was experiencing discomfort. After 5 days of treatment with the composite dressing of the present invention the surrounding tissue to the wound had greatly improved in condition, the patient was experiencing less discomfort and the wound bed was showing signs of epithelialisation. After 25 days of treatment with the composite dressing the wound had completely healed.

### Example 11

A 70 year old male patient had 3 lower limb mixed aetiology leg ulcers for approximately 2 years duration and had been treated with a wide variety of dressings (including hydrophilic foams) without success. The wounds were static. After 3 weeks treatment (changed twice a week) with a composite dressing of the present invention (gel foam, Example 3), the smallest of the wounds had completely healed and the other two had substantially reduced in size and were showing very positive signs of healing.

## Claims

1. A composition for the treatment of a wound, the composition comprising:
a first layer, which comprises a porous, optionally hydrophilic, material capable of absorbing fluid from the wound at least in part by capillary action,
a second layer comprising an absorbent hydrogel, the first layer being associated with the second layer; wherein the hydrogel comprises a hydrophilic polymer carrying multiple pendant sulphonyl groups on each polymer molecule, at least some of the pendant sulphonyl groups being present in salt form, sodium and potassium countercations being present in the hydrogel associated with the pendant groups, and wherein the molar ratio of the sodium ions to the potassium ions in the hydrogel is in the range between 100:0.1 and 100:10;
wherein, in the treatment, the first layer is disposed closer to the wound than the second layer and the composition modulates the concentration of dissolved ions in the fluid in the wound.

2. The composition for use according to claim 1, wherein the second layer has a rate of absorption of fluid that is the same as or less than the first layer.

3. The composition for use according to claim 1 or claim 2, wherein the first and second layers are laminated together and the second layer comprises a crosslinked hydrogel, wherein the hydrogel has been formed from a pregel mixture comprising one or more monomers and a crosslinking agent, and the weight:weight ratio of the total amount of monomer in the pregel mixture to the amount of crosslinking agent in the pregel mixture is from about 250:1 to about 800:1.

4. The composition for use according to any one of the preceding claims, wherein the wound is a chronic ulcerous skin lesion.

5. The composition for use according to claim 4, wherein the chronic ulcerous skin lesion is selected from venous leg ulcers, venous foot ulcers, arterial leg ulcers, arterial foot ulcers, decubitus ulcers, post-surgical ulcerous lesions and chronic burn lesions.

6. The composition for use according to any one of the preceding claims, wherein the hydrogel comprises a polymer formed from the polymerisation of the sodium salt of 2-acrylamido-2-methylpropane sulphonic acid and the potassium salt of acrylic acid (3-sulphopropyl) ester.

7. The composition for use according to any one of the preceding claims, wherein the hydrogel of the second layer is substantially non-porous.

8. The composition for use according to any one of the preceding claims, wherein the first layer comprises a hydrophilic, fibrous material and/or a hydrophilic, foamed material.

9. The composition for use according to any one of the preceding claims, wherein the first layer comprises a hydrophilic, foamed material.

10. The composition for use according to claim 9, wherein the hydrophilic, foamed material.comprises a hydrophilic polyurethane foam.

11. The composition for use according to any one of the preceding claims, wherein the composition comprises a third layer disposed on a side of the second layer away from the wound, the third layer comprising a breathable polymeric material.

12. A method of making a composition for the treatment of wounds, the method comprising
associating a first layer, which comprises a porous, optionally hydrophilic, material capable of absorbing fluid at least in part by capillary action, with a second layer comprising an absorbent hydrogel to form the composition,
wherein the hydrogel comprises a hydrophilic polymer carrying multiple pendant sulphonyl groups on each polymer molecule, at least some of the pendant sulphonyl groups being present in salt form, sodium and potassium countercations being present in the hydrogel associated with the pendant groups, and wherein the molar ratio of the sodium ions to the potassium ions in the hydrogel is in the range between 100:0.1 and 100:10.

13. The method according to claim 12, wherein the composition is as defined in any one of claims 2 to 11.

14. A wound dressing comprising:
a first layer, which comprises a porous, optionally hydrophilic material capable of absorbing fluid from the wound at least in part by capillary action,
a second layer comprising an absorbent hydrogel, the first layer being associated with the second layer; wherein the hydrogel comprises a hydrophilic polymer carrying multiple pendant sulphonyl groups on each polymer molecule, at least some of the pendant sulphonyl groups being present in salt form, sodium and potassium countercations being present in the hydrogel associated with the pendant groups, and wherein the molar ratio of the sodium ions to the potassium ions in the hydrogel is in the range between 100:0.1 and 100:10;
wherein the second layer has a rate of absorption of fluid that is the same as or less than the first layer and, in use, the first layer is disposed closer to the wound than the second layer and the dressing modulates the concentration of dissolved ions in the fluid in the wound.

15. The wound dressing according to claim 14, wherein the first and second layers and/or the wound are as defined in any one of claims 2 to 11.

## Patentansprüche

1. Zusammensetzung für die Wundbehandlung, die Zusammensetzung umfassend
eine erste Schicht, die ein poröses, wahlweise hydrophiles, Material umfasst, das in der Lage ist, zumindest teilweise durch Kapillareffekt Fluid aus der Wunde zu absorbieren,
eine zweite Schicht, umfassend ein absorbierendes Hydrogel, wobei die erste Schicht mit der zweiten Schicht verbunden ist; wobei das Hydrogel ein hydrophiles Polymer umfasst, das mehrere hängende Sulfonylgruppen an jedem Polymermolekül trägt, wobei mindestens einige der hängenden Sulfonylgruppen in Salzform vorliegen, wobei Natrium- und Kaliumgegenionen im Hydrogel vorliegen und mit den hängenden Gruppen verbunden sind, und wobei das Molarverhältnis der Natriumionen zu den Kaliumionen im Hydrogel im Bereich von 100:0,1 bis 100:10 ist;
wobei, in der Behandlung, die erste Schicht näher an der Wunde angeordnet ist als die zweite Schicht und die Zusammensetzung die Konzentration der gelösten Ionen im Fluid in der Wunde moduliert.

2. Zusammensetzung zur Verwendung gemäß Anspruch 1, wobei die zweite Schicht eine Absorptionsrate des Fluids hat, die gleich oder geringer ist als die erste Schicht.

3. Zusammensetzung zur Verwendung gemäß Anspruch 1 oder Anspruch 2, wobei die erste und die zweite Schicht zusammen laminiert sind und die zweite Schicht ein quervernetztes Hydrogel umfasst, wobei das Hydrogel gebildet worden ist aus einer Vorgelmischung, umfassend ein oder mehrere Monomere und ein Quervernetzungsmittel, und das Gewicht/Gewicht-Verhältnis der Gesamtmenge Monomer in der Vorgelmischung zur Menge Quervernetzungsmittel in der Vorgelmischung von etwa 250:1 bis etwa 800:1 ist.

4. Zusammensetzung zur Verwendung gemäß irgendeinem der vorhergehenden Ansprüche, wobei die Wunde eine chronische eiternde Hautwunde ist.

5. Zusammensetzung zur Verwendung gemäß Anspruch 4, wobei die chronische eiternde Hautwunde ausgewählt ist aus Beinvenengeschwüren, Fußvenengeschwüren, Beinarteriengeschwüren, Fußarteriengeschwüren, Druckgeschwüren, postchirurgische eiternde Hautwunden und chronische Verbrennungswunden.

6. Zusammensetzung zur Verwendung gemäß irgendeinem der vorhergehenden Ansprüche, wobei das Hydrogel ein Polymer umfasst, gebildet aus der Polymerisierung des Natriumsalzes der 2-Acrylamido-2-methylpropansulfonsäure und des Kaliumsalzes des Acrylsäure(3-sulfopropyl)esters.

7. Zusammensetzung zur Verwendung gemäß irgendeinem der vorhergehenden Ansprüche, wobei das Hydrogel der zweiten Schicht im Wesentlichen nicht porös ist.

8. Zusammensetzung zur Verwendung gemäß irgendeinem der vorhergehenden Ansprüche, wobei die erste Schicht ein hydrophiles faseriges Material umfasst und/oder ein hydrophiles aufgeschäumtes Material.

9. Zusammensetzung zur Verwendung gemäß irgendeinem der vorhergehenden Ansprüche, wobei die erste Schicht ein hydrophiles aufgeschäumtes Material umfasst.

10. Zusammensetzung zur Verwendung gemäß Anspruch 9, wobei das hydrophile aufgeschäumte Material einen hydrophilen Polyurethanschaum umfasst.

11. Zusammensetzung zur Verwendung gemäß irgendeinem der vorhergehenden Ansprüche, wobei die Zusammensetzung eine dritte Schicht umfasst, angeordnet an einer von der Wunde abgelegenen Seite der zweiten Schicht, wobei die dritte Schicht ein atmungsfähiges polymerisches Material umfasst.

12. Herstellungsverfahren für eine Zusammensetzung für die Wundbehandlung, das Verfahren umfassend
Verbinden einer ersten Schicht, die ein poröses, wahlweise hydrophiles, Material umfasst, das in der Lage ist, zumindest teilweise durch Kapillareffekt Fluid zu absorbieren, mit einer zweiten Schicht, umfassend ein absorbierendes Hydrogel zum Bilden der Zusammensetzung,
wobei das Hydrogel ein hydrophiles Polymer umfasst, das mehrere hängende Sulfonylgruppen an jedem Polymermolekül trägt, wobei mindestens einige der hängenden Sulfonylgruppen in Salzform vorliegen, wobei Natrium- und Kaliumgegenionen im Hydrogel vorliegen und mit den hängenden Gruppen verbunden sind, und wobei das Molarverhältnis der Natriumionen zu den Kaliumionen im Hydrogel im Bereich von 100:0,1 bis 100:10 ist

13. Verfahren gemäß Anspruch 12, wobei die Zusammensetzung wie in irgendeinem der Ansprüche 2 bis 11 definiert ist.

14. Wundverband, umfassend
eine erste Schicht, welche ein poröses, wahlweise hydrophiles, Material umfasst, das in der Lage ist, zumindest teilweise durch Kapillareffekt Fluid aus der Wunde zu absorbieren,
eine zweite Schicht, umfassend ein absorbierendes Hydrogel, wobei die erste Schicht mit der zweiten Schicht verbunden ist; wobei das Hydrogel ein hydrophiles Polymer umfasst, das mehrere hängende Sulfonylgruppen an jedem Polymermolekül trägt, wobei mindestens einige der hängenden Sulfonylgruppen in Salzform vorliegen, wobei Natrium- und Kaliumgegenionen im Hydrogel vorliegen und mit den hängenden Gruppen verbunden sind, und wobei das Molarverhältnis der Natriumionen zu den Kaliumionen im Hydrogel im Bereich von 100:0,1 bis 100:10 ist;
wobei die zweite Schicht eine Absorptionsrate für Fluid hatdie gleich oder geringer als die erste Schicht ist und, in der Behandlung, die erste Schicht näher an der Wunde angeordnet ist als die zweite Schicht und der Verband die Konzentration der gelösten Ionen im Fluid der Wunde moduliert.

15. Wundverband gemäß Anspruch 14, wobei die erste und die zweite Schicht und/oder die Wunde wie in irgendeinem der Ansprüche 2 bis 11 definiert sind.

## Revendications

1. Une composition pour le traitement d'une plaie, la composition comprenant :
une première couche, qui contient un matériau poreux éventuellement hydrophile capable d'absorber un fluide provenant de la plaie au moins en partie par action capillaire,
une deuxième couche contenant un hydrogel absorbant, la première couche étant associée à la deuxième couche, où l'hydrogel contient un polymère hydrophile transportant une pluralité de groupes sulfonyle pendants sur chaque molécule polymère, au moins certains des groupes sulfonyle pendants étant présents sous forme saline, des contre-cations de potassium et de sodium étant présents dans l'hydrogel associé aux groupes pendants, et où le rapport molaire des ions sodium aux ions potassium dans l'hydrogel se situe dans la plage entre 100:0,1 et 100:10,
où, dans le traitement, la première couche est disposée plus proche de la plaie que la deuxième couche et la composition module la concentration d'ions dissous dans le fluide dans la plaie.

2. La composition destinée à une utilisation selon la revendication 1, où la deuxième couche possède un taux d'absorption de fluide qui est le même que ou inférieur à celui de la première couche.

3. La composition destinée à une utilisation selon la revendication 1 ou 2, où les première et deuxième couches sont laminées ensemble et la deuxième couche contient un hydrogel réticulé, où l'hydrogel a été formé à partir d'un mélange de prégel contenant un ou plusieurs monomères et un agent de réticulation, et le rapport poids:poids de la quantité totale de monomère dans le mélange de prégel sur la quantité d'agent de réticulation dans le mélange de prégel est d'environ 250:1 à environ 800:1.

4. La composition destinée à une utilisation selon l'une quelconque des revendications précédentes, où la plaie est une lésion cutanée ulcéreuse chronique.

5. La composition destinée à une utilisation selon la revendication 4, où la lésion cutanée ulcéreuse chronique est sélectionnée parmi ulcères veineux de la jambe, ulcères veineux du pied, ulcères artériels de la jambe, ulcères artériels du pied, ulcères de décubitus, lésions ulcéreuses post-chirurgicales et lésions par brûlures chroniques.

6. La composition destinée à une utilisation selon l'une quelconque des revendications précédentes, où l'hydrogel contient un polymère formé à partir de la polymérisation du sel de sodium d'un acide sulfonique 2-acrylamido-2-méthylepropane et du sel de potassium d'un ester d'acide acrylique (3-sulfopropyle).

7. La composition destinée à une utilisation selon l'une quelconque des revendications précédentes, où l'hydrogel de la deuxième couche est sensiblement non poreux.

8. La composition destinée à une utilisation selon l'une quelconque des revendications précédentes, où la première couche contient un matériau fibreux hydrophile et/ou un matériau en mousse hydrophile.

9. La composition destinée à une utilisation selon l'une quelconque des revendications précédentes, où la première couche contient un matériau en mousse hydrophile.

10. La composition destinée à une utilisation selon la revendication 9, où le matériau en mousse hydrophile contient une mousse polyuréthane hydrophile.

11. La composition destinée à une utilisation selon l'une quelconque des revendications précédentes, où la composition contient une troisième couche disposée sur un côté de la deuxième couche à l'écart de la plaie, la troisième couche contenant un matériau polymère perméable à l'air.

12. Un procédé de fabrication d'une composition pour le traitement de plaies, le procédé comprenant
l'association d'une première couche, qui contient un matériau poreux éventuellement hydrophile capable d'absorber un fluide au moins en partie par action capillaire, avec une deuxième couche contenant un hydrogel absorbant de façon à former la composition,
où l'hydrogel contient un polymère hydrophile transportant une pluralité de groupes sulfonyle pendants sur chaque molécule polymère, au moins certains des groupes sulfonyle pendants étant présents sous forme saline, des contre-cations de potassium et de sodium étant présents dans l'hydrogel associé aux groupes pendants, et où le rapport molaire des ions sodium aux ions potassium dans l'hydrogel se situe dans la plage entre 100:0,1 et 100:10.

13. Le procédé selon la revendication 12, où la composition est telle que définie dans l'une quelconque des revendications 2 à 11.

14. Un pansement de plaie comprenant :
une première couche, qui contient un matériau poreux éventuellement hydrophile capable d'absorber un fluide provenant de la plaie au moins en partie par action capillaire,
une deuxième couche contenant un hydrogel absorbant, la première couche étant associée à la deuxième couche, où l'hydrogel contient un polymère hydrophile transportant une pluralité de groupes sulfonyle pendants sur chaque molécule polymère, au moins certains des groupes sulfonyle pendants étant présents sous forme saline, des contre-cations de potassium et de sodium étant présents dans l'hydrogel associé aux groupes pendants, et où le rapport molaire des ions sodium aux ions potassium dans l'hydrogel se situe dans la plage entre 100:0,1 et 100:10,
où la deuxième couche possède un taux d'absorption de fluide qui est le même que ou inférieur à celui de la première couche et, en utilisation, la première couche est disposée plus proche de la plaie que la deuxième couche et le pansement module la concentration d'ions dissous dans le fluide dans la plaie.

15. Le pansement de plaie selon la revendication 14, où les première et deuxième couches et/ou la plaie sont telles que dans l'une quelconque des revendications 2 à 11.
